# EUROPEAN PATENT APPLICATION

(11) **EP 2 962 995 A1**
(43) Date of publication of application: **06.01.2016**
(21) Application number: 15171761.8
(22) Date of filing: 12.06.2015
(51) Int. Cl.: C01G 25/00, G01N 27/407, G01N 33/00, H01M 8/12

(54) **PROTON-CONDUCTING OXIDE**

(30) Priority: 01.07.2014 JP 2014136042
(71) Applicant: Panasonic Intellectual Property Management Co., Ltd., Osaka-shi, Osaka 540-6207 (JP)
(72) Inventor: TAKEUCHI, Hiroki, Chuo-ku, Osaka 540-6207 (JP); ZENITANI, Yuji, Chuo-ku, Osaka 540-6207 (JP); ASANO, Tetsuya, Chuo-ku, Osaka 540-6207 (JP)
(74) Representative: Grünecker Patent- und Rechtsanwälte PartG mbB

(57) **Abstract**

The present invention provides a proton-conducting oxide comprising a perovskite crystal structure represented by a composition formula AₐB₁₋ₓB'ₓO_{3-δ}. A represents strontium. B represents zirconium. B' represents at least one selected from the group consisting of yttrium and ytterbium. The value of a is more than 0.84 and less than 1.0. The value of x is more than 0.0 and less than 0.2. The present invention provides a proton-conducting oxide having a capability to maintain high proton conductivity even if the proton-conducting oxide is exposed to the air atmosphere for a long time.

## Description

### BACKGROUND

### 1. Technical Filed

The present invention relates to a proton-conducting oxide.

### 2. Description of the Related Art

Japanese Patent Publication No. 4634252 discloses an oxide having a perovskite crystal structure represented by the composition formula AₐB₁₋ₓB'ₓO_{3-δ} (0.5 < a < 2.0, 0 < x < 0.2, A is an element selected from the group consisting of Ba, Mg, Ca and Sr, B is an element selected from the group consisting of Ce, Zr, Ti and Hf, and B' is an element selected from the group consisting of Group 3 elements and Group 13 elements.

### SUMMARY

The present invention provides a proton-conducting oxide comprising:
a perovskite crystal structure represented by a composition formula AₐB₁₋ₓB'ₓO_{3-δ}, wherein
A represents strontium;
B represents zirconium;
B' represents at least one selected from the group consisting of yttrium and ytterbium;
a is more than 0.84 and less than 1.0; and
x is more than 0.0 and less than 0.2.

The present invention provides a proton-conducting oxide having a capability to maintain high proton conductivity even if the proton-conducting oxide is exposed to the air atmosphere for a long time.

### BRIEF DESCRIPTION OF THE DRAWINGS

FIG. 1 is a graph showing a proton conductivity of the proton-conducting oxide according to the inventive example 1 within a range of the temperature of 100 degrees Celsius - 300 degrees Celsius.
FIG. 2 is a graph showing proton conductivities of the proton-conducting oxides according to the inventive example 1 and the comparative example 2 at a temperature of 300 degrees Celsius under a saturated water vapor atmosphere.
FIG. 3 shows a cross-sectional view of a device comprising the proton-conducting oxide according to the present invention.

### DETAILED DESCRIPTION OF THE EMBODIMENT

Japanese Patent Publication No. 4634252 fails to disclose proton conductivity of the proton-conducting oxide exposed to the air atmosphere. The present invention provides a proton-conducting oxide having a capability to maintain high proton conductivity even if the proton-conducting oxide is exposed to the air atmosphere for a long time.

### (First embodiment)

Hereinafter, a proton-conducting oxide according to the first embodiment will be described. The proton-conducting oxide according to the first embodiment is a metal oxide having a perovskite crystal structure represented by the composition formula AₐB₁₋ₓB'ₓO_{3-δ}.

### <Element A>

The element A is strontium (i.e., Sr). The value of "a" in the composition formula AₐB₁₋ₓB'ₓ0_{3-δ} is more than 0.84 and less than 1.0. The value of "a" indicates the element content of A. More specifically, the value of "a" is not less than 0.87 and not more than 0.97.

### <Element B>

The element B is zirconium (i.e., Zr). The element B of zirconium allows the perovskite structure to be stable. For this reason, the component which does not have proton conductivity is hardly generated in the proton-conducting oxide according to the first embodiment. As a result, a proton-conducting oxide having high proton conductivity is obtained, and therefore zirconium is desirable. The value of "x" in the composition formula AₐB₁₋ₓB'ₓO_{3-δ} is more than 0.0 and less than 0.2. The value of "b" indicates the element content of B. More specifically, the value of "b" is not less than 0.02 and not more than 0.18.

### <Element B'>

The element B' is selected from the group consisting of yttrium (i.e., Y) and ytterbium (i.e., Yb). It is desirable that the element B' has an ion radius of more than 0.05 nanometers and less than 0.102 nanometers. Such an element B' allows the perovskite structure to maintain stability. As a result, a proton-conducting oxide having high proton conductivity is obtained.

It is desirable that B' is yttrium (i.e., Y), since the proton-conducting oxide having yttrium has a stable perovskite structure and high proton conductivity.

### (Regarding "δ")

The value of "δ" may be more than 0 and less than 3. As one example, the value of "δ" is more than 2.5 and less than 3. The value of "δ" indicates the element content of O (i.e., oxygen).

Desirably, the proton-conducting oxide according to the first embodiment has a flat surface.

### (Fabrication method)

The proton-conducting oxide according to the first embodiment can be formed by a sputtering method, a plasma laser deposition method (hereinafter, referred to as "PLD method"), or a chemical vapor deposition method (hereinafter, referred to as "CVD method"). The fabrication method of the proton-conducting oxide is not limited. Alternatively, the proton-conducting oxide can be formed by a solid reaction method or a hydrothermal synthesis method.

### (Others)

In the present specification, the proton-conducting oxide according to the first embodiment may be referred to as a "proton conductor". The proton-conducting oxide may have a shape of a film.

The proton-conducting oxide according to the first embodiment may be formed on a substrate. An example of the material of the substrate is magnesium oxide represented by the chemical formula MgO, strontium titanate represented by the chemical formula SrTiO₃, or silicon represented by the chemical formula Si. If the proton-conducting oxide according to the first embodiment is used for a fuel cell, at least a part of the substrate may be removed.

The proton-conducting oxide according to the first embodiment may be monocrystalline or polycrystalline. Desirable is the proton-conducting oxide having a crystal oriented by controlling an orientation of the crystal growth on a magnesium oxide substrate (i.e., MgO substrate), a strontium titanate substrate (i.e., SrTiO₃ substrate), a silicon substrate (i.e., Si substrate) which has a buffer layer of which lattice constant is controlled, since the proton-conducting oxide has higher proton conductivity. The proton-conducting oxide having a monocrystalline structure epitaxially grown on the substrate is also desirable, since the proton-conducting oxide has much higher proton conductivity. Such a monocrystalline structure may be obtained by appropriately selecting the surface orientation of the substrate, and film forming conditions such as temperature, pressure, and atmosphere. However, neither the requirement of the formation of the film nor the crystal system is limited.

As known conventionally, the tetravalent element B contained in the proton-conducting oxide having a perovskite structure may be substituted with the trivalent element B' to generate oxygen defects in the proton-conducting oxide. Water molecules (i.e., H₂O) are introduced in the oxygen defects. For this reason, carriers of protons are introduced with regard to the proton-conducting oxide.

Thus, protons migrate in a hopping conduction way around the oxygen molecules contained in the proton-conducting oxide. In other words, the proton-conducting oxide exhibits the proton conductivity. The temperature dependency of the proton conductivity is a thermally-activated dependency having an activation energy of about 0.4 eV - 1.0 eV. For this reason, the proton conductivity decreases exponentially with a decrease in the temperature.

In order to increase the proton conductivity, it is conventionally proposed to increase an amount of protons which are carriers. In a perovskite proton-conducting oxide, the tetravalent element B is substituted with the tetravalent element B' to introduce the oxygen defects in the crystal structure. The oxygen defects are introduced in light of the difference between the valences of the tetravalent and trivalent elements in such a manner that electrical neutrality is maintained. The water molecules (i.e., H₂O) are introduced in the thus-introduced oxygen defects. In this way, protons are injected into the oxide to exhibit the proton conductivity.

However, an upper limit of the element content of B' is approximately 0.2 in the conventional perovskite proton-conducting oxide. As just described, there is an upper limit in the amount of the oxygen defects.

In addition, as a method for introducing a larger amount of the proton carriers, a decrease in the element content of A is expected to have a similar effect to the case where the element content of B' is increased. However, when the value of "a" is less than 1, the proton conductivity is lowered. It is believed that the reason therefor is that a component having no proton conductivity is generated in the crystal.

When the activation energy of the proton conductivity is not more than 0.1 eV, the high proton conductivity of the proton-conducting oxide is maintained at a value of not less than 10⁻² S/cm even within a temperature range of 100 degrees Celsius - 300 degrees Celsius. Thus, it is desirable that the decrease of the proton conductivity due to the decrease in the temperature is prevented.

In the perovskite proton-conducting oxide represented by the composition formula AₐB₁₋ₓB'ₓO_{3-δ}, the value of "a" is not less than 0.87 and not more than 0.97, and the value of "x" is not less than 0.05 and not more than 0.18. Such values of "a" and "x" allow protons (i.e., hydrogen ions) to be easily introduced in the sample and the activation energy to be lower than the proton conductivity of the conventional proton-conducting oxide.

### (Findings by the present inventors)

Furthermore, the present inventors found that the proton-conducting oxide having strontium (i.e., Sr) as the element A has higher durability than the one having barium (i.e., Ba) as the element A, since the proton conductivity of the proton-conducting oxide having strontium (i.e., Sr) is not so lowered in a case where it is exposed to the air atmosphere.

Since strontium has a smaller ion radius and a smaller oxygen coordination number than barium, strontium prevents an excess amount of oxygen from being introduced in the crystal structure. For this reason, it is believed that the proton-conducting oxide having strontium (i.e., Sr) as the element A has high durability.

As a result, obtained is a perovskite proton-conducting oxide having high proton conductivity and high durability in the air atmosphere.

Since the proton-conducting oxide according to the first embodiment has low activation energy, it has both high proton conductivity and durability within all the temperature range (i.e., less than 100 degrees Celsius and more than 300 degrees Celsius).

### EXAMPLES

Hereinafter, the present invention will be described in more detail with reference to the following examples.

### (Inventive example 1)

A substrate having a size of 10 mm x 10 mm and a thickness of 0.5 mm was put on a substrate holder having a heater. The substrate holder was included in a vacuum chamber. Then, the vacuum chamber was decompressed to approximately 10⁻³ Pa. The material of the substrate was monocrystalline magnesium oxide (i.e., MgO).

The substrate was heated with the heater to 650 degrees Celsius to 750 degrees Celsius. An oxygen gas having a flow rate of 2 sccm and an argon gas having a flow rate of 8 sccm were introduced in the vacuum chamber. In this way, the pressure in the vacuum chamber was adjusted to approximately 1 Pa.

A film of a proton-conducting oxide was formed on the substrate by a sputtering method using a sintered target having a Sr:Zr:Y element ratio of 10:8:2.

The composition ratio and the proton conductivity of the thus-formed proton-conducting oxide were evaluated. Table 1 shows the results. Hereinafter, the evaluation methods and the results thereof will be described.

The proton-conducting oxide was subjected to an X-ray diffraction analysis using a Cu target. The present inventors confirmed that the obtained proton-conducting oxide had a perovskite crystal structure.

The composition ratio of the obtained proton-conducting oxide was analyzed using an electron probe micro analyzer (EPMA). As shown in Table 1, it was revealed that the obtained proton-conducting oxide having a composition formula AₐB₁₋ₓB'ₓ0_{3-δ} had an element A of strontium (i.e., Sr) and that the values of "a" and "x" were 0.87 and 0.18, respectively.

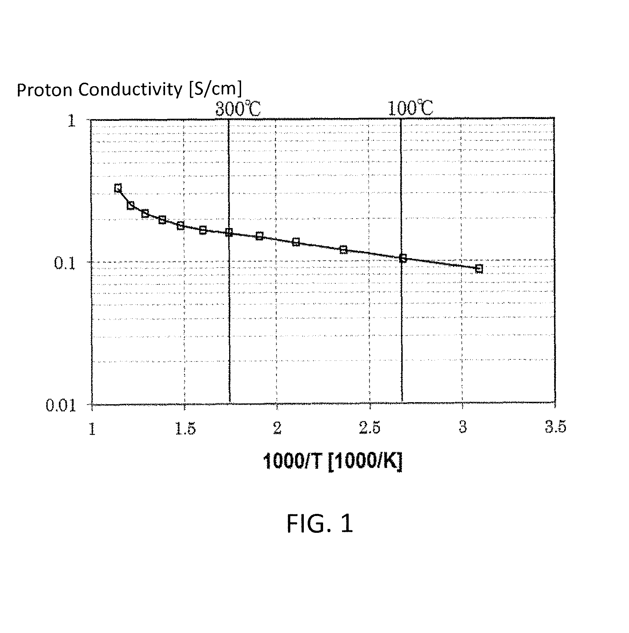

The proton conductivity of the obtained proton-conducting oxide was measured by an impedance method within a temperature range of 50 degrees Celsius to 600 degrees Celsius in an argon gas having hydrogen at a concentration of 5%. FIG. 1 shows temperature dependency of the proton conductivity.

### (Durability test)

A conventional proton-conducting perovskite oxide exhibits proton conductivity under a humidified atmosphere. However, the present inventors found that the proton conductivity of the proton-conducting perovskite oxide AₐB₁₋ₓB'ₓ0_{3-δ} is decreased significantly under a humidified atmosphere in a case where A is barium, even when the proton-conducting perovskite oxide exhibits a proton conductivity of not less than 10⁻² S/cm within a temperature range of 100 degrees Celsius - 300 degrees Celsius. Therefore, the present inventors put the proton-conducting perovskite oxide to the following test to evaluate its durability against water included in an atmosphere as below.

The proton-conducting oxide according to the inventive example 1 was put in a water vapor atmosphere at a temperature of 300 degrees Celsius. The conductivity of the proton-conducting oxide was measured by an alternating-current impedance method at regular time intervals to evaluate its durability.

Specifically, the water vapor atmosphere was made by bubbling water using an argon gas at a temperature of 25 degrees Celsius. Then, a pair of silver electrodes were formed at both ends of the proton-conducting oxide according to the inventive example 1. The conductivity thereof was measured by an impedance method to evaluate its durability. Table 1 shows the results.

As shown in Table 1, the proton conductivity at 100 degrees Celsius was 0.103 S/cm, and that at 300 degrees Celsius was 0.158 S/cm. The proton conductivity was 0.155 S/cm after the durability test. The decrease ratio of the proton conductivity was 2%. In this way, the present inventors confirmed that the proton-conducting oxide according to the inventive example 1 was stable even under an oxidation atmosphere.

### (Inventive example 2)

An experiment similar to the inventive example 1 was conducted, except that the proton-conducting oxide was formed using a sintered target having a Sr:Zr:Y element ratio of 10:9:1. The results are shown in Table 1.

The present inventors confirmed that the proton-conducting oxide according to the inventive example 2 had a perovskite crystal structure. As shown in Table 1, it was revealed that the obtained proton-conducting oxide AₐB₁₋ₓB'ₓO_{3-δ} according to the inventive example 2 had an element A of strontium (i.e., Sr) and that the value of "a" was 0.91. Further, the obtained proton-conducting oxide AₐB₁₋ₓB'ₓO_{3-δ} had elements B and B' of zirconium (i.e., Zr) and yttrium (i.e., Y) respectively and the value of "x" was 0.10. As shown in Table 1, the proton conductivity at 100 degrees Celsius was 0.107 S/cm, and that at 300 degrees Celsius was 0.174 S/cm. The proton conductivity was 0.172 S/cm after the durability test. The decrease ratio of the proton conductivity was 1%. In this way, the present inventors confirmed that the proton-conducting oxide according to the inventive example 2 was stable even under an oxidation atmosphere.

### (Inventive example 3)

An experiment similar to the inventive example 1 was conducted, except that the proton-conducting oxide was formed using a sintered target having a Sr:Zr:Y element ratio of 10:10:0.5. The results are shown in Table 1.

The present inventors confirmed that the proton-conducting oxide according to the inventive example 3 had a perovskite crystal structure. As shown in Table 1, it was revealed that the obtained proton-conducting oxide AₐB₁₋ₓB'ₓ0_{3-δ} according to the inventive example 3 had an element A of strontium (i.e., Sr) and that the value of "a" was 0.97. Further, the obtained proton-conducting oxide AₐB₁₋ₓB'ₓO_{3-δ} had elements B and B' of zirconium (i.e., Zr) and yttrium (i.e., Y) respectively and the value of "x" was 0.02. As shown in Table 1, the proton conductivity at 100 degrees Celsius was 0.085 S/cm, and that at 300 degrees Celsius was 0.140 S/cm. The proton conductivity was 0.137 S/cm after the durability test. The decrease ratio of the proton conductivity was 2%. In this way, the present inventors confirmed that the proton-conducting oxide according to the inventive example 3 was stable even under an oxidation atmosphere.

### (Inventive example 4)

An experiment similar to the inventive example 1 was conducted, except that the proton-conducting oxide was formed using a sintered target having a Sr:Zr:Yb element ratio of 10:9:1. The results are shown in Table 1.

The present inventors confirmed that the proton-conducting oxide according to the inventive example 4 had a perovskite crystal structure. As shown in Table 1, it was revealed that the obtained proton-conducting oxide AₐB₁₋ₓB'ₓ0_{3-δ} according to the inventive example 4 had an element A of strontium (i.e., Sr) and that the value of "a" was 0.97. Further, the obtained proton-conducting oxide AₐB₁₋ₓB'ₓO_{3-δ} had elements B and B' of zirconium (i.e., Zr) and ytterbium (i.e., Yb) respectively and the value of "x" was 0.05. As shown in Table 1, the proton conductivity at 100 degrees Celsius was 0.098 S/cm, and that at 300 degrees Celsius was 0.179 S/cm. The proton conductivity was 0.175 S/cm after the durability test. The decrease ratio of the proton conductivity was 2%. In this way, the present inventors confirmed that the proton-conducting oxide according to the inventive example 4 was stable even under an oxidation atmosphere.

### (Comparative example 1)

In the comparative example 1, a proton-conducting oxide AₐB₁₋ₓB'ₓO_{3-δ} having an element A of barium (i.e., Ba) was fabricated similarly in order to compare the stability of the proton conductivity under a long-time exposure to the air atmosphere. Specifically, an experiment similar to the inventive example 1 was conducted, except that the proton-conducting oxide was formed using a sintered target having a Ba:Zr:Ce:Nd element ratio of 10:5:4:1. The results are shown in Table 1.

The present inventors confirmed that the proton-conducting oxide according to the comparative example 1 had a perovskite crystal structure. As shown in Table 1, it was revealed that the obtained proton-conducting oxide AₐB₁₋ₓB'ₓO_{3-δ} according to the comparative example 1 had an element A of barium (i.e., Ba) and that the value of "a" was 0.98. Further, the obtained proton-conducting oxide AₐB₁₋ₓB'ₓO_{3-δ} had an element B of zirconium (i.e., Zr) and cerium (i.e., Ce). The values of zirconium and cerium were 0.50 and 0.41, respectively. The element B' was neodymium (i.e., Nd). The value of "x" was 0.10. As shown in Table 1, the proton conductivity at 100 degrees Celsius was 0.334 S/cm, and that at 300 degrees Celsius was 0.430 S/cm. The proton conductivity was 0.387 S/cm after the durability test. The decrease ratio of the proton conductivity was 10%. In this way, the present inventors confirmed that the proton-conducting oxide according to the comparative example 1 was deteriorated under an oxidation atmosphere.

### (Comparative example 2)

In the comparative example 2, an experiment similar to the inventive example 1 was conducted, except that the proton-conducting oxide was formed using a sintered target having a Ba:Zr:Y element ratio of 10:8:2. The results are shown in Table 1.

The present inventors confirmed that the proton-conducting oxide according to the comparative example 2 had a perovskite crystal structure. As shown in Table 1, it was revealed that the obtained proton-conducting oxide AₐB₁₋ₓB'ₓO_{3-δ} according to the comparative example 2 had an element A of barium (i.e., Ba) and that the value of "a" was 0.70. Further, the obtained proton-conducting oxide AₐB₁₋ₓB'ₓ0_{3-δ} had elements B and B' of zirconium (i.e., Zr) and yttrium (i.e., Y) respectively and the value of "x" was 0.19. As shown in Table 1, the proton conductivity at 100 degrees Celsius was 0.783 S/cm, and that at 300 degrees Celsius was 1.07 S/cm. The proton conductivity was 0.920 S/cm after the durability test. The decrease ratio of the proton conductivity was 14%. In this way, the present inventors confirmed that the proton-conducting oxide according to the comparative example 2 was deteriorated under an oxidation atmosphere.

As shown in Table 1, the proton-conducting oxides according to the inventive examples 1 - 4 and the comparative examples 1 - 2 had a high proton conductivity of not less than 10⁻² S/cm within a temperature range of 100 degrees Celsius - 300 degrees Celsius.

**[Table 1]**

| Sample | A | a | B' | X | Conductivity (S/cm) | | | Decrease ratio of conductivity (%) |
|---|---|---|---|---|---|---|---|---|
| | | | | | Before the durability test | | After the durability test | |
| | | | | | 100 degrees Celsius | 300 degrees Celsius | 300 degrees Celsius | |
| I.E. 1 | Sr | 0.87 | Y | 0.18 | 0.103 | 0.158 | 0.155 | 2 |
| I.E. 2 | Sr | 0.91 | Y | 0.10 | 0.107 | 0.174 | 0.172 | 1 |
| I.E. 3 | Sr | 0.97 | Y | 0.02 | 0.085 | 0.140 | 0.137 | 2 |
| I.E. 4 | Sr | 0.97 | Yb | 0.05 | 0.098 | 0.179 | 0.175 | 2 |
| C.E. 1 | Ba | 0.98 | Nd | 0.10 | 0.334 | 0.430 | 0.387 | 10 |
| C.E. 2 | Ba | 0.70 | Y | 0.19 | 0.783 | 1.07 | 0.920 | 14 |

| | | | | | | | | |
|---|---|---|---|---|---|---|---|---|
| I.E.: Inventive example C.E.: Comparative example | | | | | | | | |

The proton-conducting oxides according to the inventive examples 1 - 4 have a value of "a" of not less than 0.87 and not more than 0.97. Further, the value of "x" is not less than 0.02 and not more than 0.18. In light of the fabrication method, the composition of the proton-conducting oxide has an error ratio of approximately 5%.

As just described, the proton-conducting oxides according to the inventive examples 1 - 4 and the comparative examples 1 - 2 each have a value of "a" of more than 0.84 and less than 1.0 and a value of "x" of more than 0.0 and less than 0.2. However, Table 1 reveals that the proton-conducting oxides according to the inventive examples 1 - 4 each having an element A of strontium (i.e., Sr) have higher durability than ones according to the comparative examples 1 - 2 each having an element A of barium (i.e., Ba). In other words, a proton-conducting oxide has not only high proton conductivity but also high durability, if the proton-conducting oxide has an element A of strontium (i.e., Sr), a value of "a" of more than 0.84 (=0.87-0.03) and less than 1.0 (=0.97+0.03), and a value of "x" of more than 0.0 (=0.02-0.02) and less than 0.2 (=0.18+0.02).

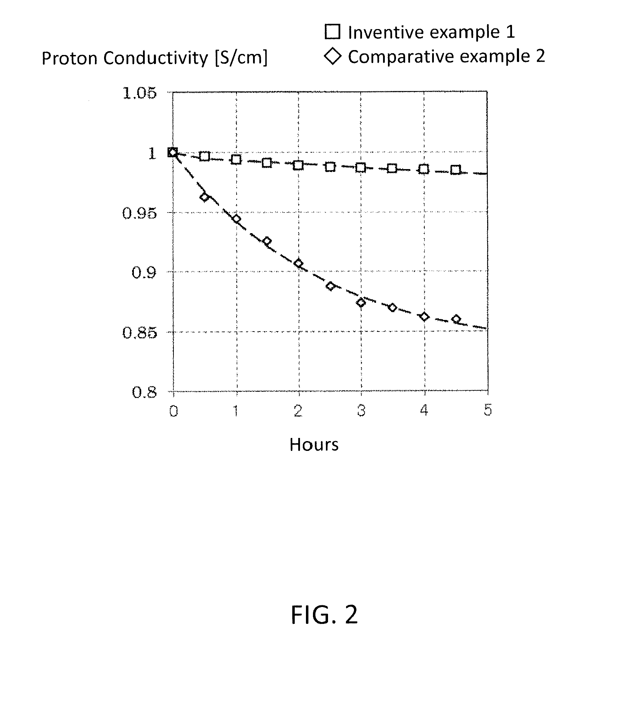

FIG. 2 shows how the proton conductivities of the proton-conducting oxides according to the inventive example 1 and the comparative example 2 were changed when the proton-conducting oxides were exposed to a saturated water vapor atmosphere at 300 degrees Celsius. The saturated water vapor atmosphere at 300 degrees Celsius is a severer oxidation atmosphere than the air atmosphere. Therefore, the saturated water vapor atmosphere can cut the time for the evaluation of the stability of the proton conductivity.

The proton-conducting oxide according to the inventive example 1 having an element A of strontium (i.e., Sr) was hardly deteriorated even after exposed to the saturated water vapor atmosphore. The conductivity decrease ratio thereof was 2%. On the other hand, the proton-conducting oxide according to the comparative example 2 having an element A of barium (i.e., Ba) was significantly deteriorated after the exposure just for five hours. The conductivity decrease ratio thereof was 14%.

These results reveal that the proton-conducting oxide having an element A of strontium (i.e., Sr) which has a smaller ion radius and a smaller oxygen coordination number than barium has high stability, since excess oxygen atoms are hardly incorporated in the crystal structure thereof.

By using the proton-conducting oxide according to the present invention for a conventional device, provided is a device in which its proton conductivity is not significantly decreased even after exposed to the air atmosphere for a long time.

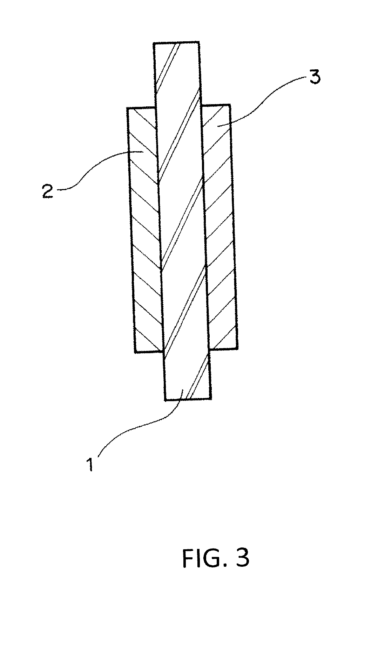

FIG. 3 shows an example of a device including the proton-conducting oxide. The device shown in FIG. 3 comprises a proton-conducting oxide 1, an anode electrode 2, and a cathode electrode 3. An example of the device is a fuel cell, a hydrogen sensor, a water vapor electrolysis device, a hydrogen addition device, or a hydrogen desorption device. A fuel cell may be an organic hydride reproduce-type fuel cell. Such a device including a proton-conducting oxide is known. Therefore, the description thereof is omitted.

### INDUSTRIAL APPLICABILITY

The proton-conducting oxide according to the present invention can be used for a device relating to hydrogen energy such as a fuel cell, a hydrogen sensor, a water vapor electrolysis device, a hydrogen addition device, or a hydrogen desorption device that has a structure in which the proton-conducting oxide is sandwiched between an anode electrode and a cathode electrode. An example of a fuel cell using a proton-conducting oxide is disclosed in United States Patent No. 7,141,327, which is incorporated herein by reference. An example of a hydrogen sensor using a proton-conducting oxide is disclosed in United States Patent No. 7,235,171, which is incorporated herein by reference.

### REFERENCE SIGNS LIST

1: Proton-conducting oxide
2: Anode electrode
3: Cathode electrode

## Claims

1. A proton-conducting oxide comprising:
a perovskite crystal structure represented by a composition formula AₐB₁₋ₓB'ₓO_{3-δ}, wherein
A represents strontium;
B represents zirconium;
B' represents at least one selected from the group consisting of yttrium and ytterbium;
a is more than 0.84 and less than 1.0; and
x is more than 0.0 and less than 0.2.

2. A fuel cell comprising the proton-conducting oxide according to claim 1.

3. A hydrogen sensor comprising the proton-conducting oxide according to claim 1.
